# EUROPEAN PATENT APPLICATION

(11) **EP 4 414 071 A1**
(43) Date of publication of application: **14.08.2024**
(21) Application number: 22940693.9
(22) Date of filing: 20.06.2022
(51) Int. Cl.: B01J 27/135, B01J 27/18, B01J 27/10, C07D 307/46

(54) **COMPOSITE CATALYST AND SYNTHESIS METHOD FOR BIO-BASED FURAN CHEMICALS**

(30) Priority: 05.05.2022 CN 202210490575
(71) Applicant: Ningbo Institute of Materials Technology & Engineering, Chinese Academy of Sciences, Ningbo, Zhejiang 315201 (CN)
(72) Inventor: ZHANG, Jian, Ningbo, Zhejiang 315201 (CN); FENG, Sufei, Ningbo, Zhejiang 315201 (CN); LI, Mingfu, Ningbo, Zhejiang 315201 (CN); CHEN, Hui, Ningbo, Zhejiang 315201 (CN); HUAI, Liyuan, Ningbo, Zhejiang 315201 (CN); YANG, Jie, Ningbo, Zhejiang 315201 (CN)
(74) Representative: Bayramoglu et al.
(86) International application number: PCT/CN2022/099816
(87) International publication number: WO 2023/212999

(57) **Abstract**

The present application discloses a composite catalyst and a method for synthesizing biobased furan chemicals. The composite catalyst comprises tetravalent metal salts and acidic metal salts. The method for synthesizing biobased furan chemicals comprises: reacting a reaction system comprising biomass carbohydrates, the composite catalyst and a solvent. In the present application, by forming the composite catalyst with tetravalent metal salts and acidic metal salts, biomass carbohydrates can be simply and efficiently catalyzed into 5-hydroxymethylfurfural under mild conditions, and the yield of a target product is high. At the same time, the catalyst is simple in composition, easily available in raw material and has good industrial prospect.

## Description

### CROSS-REFERENCE TO RELATED APPLICATION

The present application claims the priority of Chinese Patent application with application number 202210490575.0 submitted on May 5, 2022, entitled "COMPOSITE CATALYST AND METHOD FOR SYNTHESIZING BIOBASED FURAN CHEMICALS".

### TECHNICAL FIELD

The present application relates to a composite catalyst and a method for synthesizing biobased furan chemicals, belonging to the technical field of organic chemistry.

### BACKGROUND

The biomass carbohydrate, as a renewable carbon source, is a potential fossil energy substitute, and is one of raw materials for producing biobased chemicals. One important source of the biomass carbohydrate is a marine biomass. The marine biomass is rich in reserves, short in growth period, simple in structure and easy to depolymerize, and the carbohydrate therein is high in content and easy to extract, and is a promising biomass resource. 5-hydroxymethylfurfural (5-HMF) is one of the key intermediate chemicals derived by carbohydrate dehydration, which is a versatile platform compound and can be used to synthesize various chemicals and fuels. In the process of synthesizing 5-HMF using biomass, metal salt catalysts are widely applied. Currently, the commonly used metal salt catalysts are mainly based on metal chlorides. Where, monovalent metal chlorides difficultly catalyze the conversion of biomass into furan chemicals due to their low activity. Divalent or trivalent metal salts can form complexes with sugar to promote the isomerization of sugar, which promotes the generation of 5-HMF to some extents. However, due to its low surface electron density, limited electron transfer activity, high general catalytic temperature and long time of the reaction, the efficiency of synthesizing 5-HMF from sugar biomass is limited (for example CN109111414A). How to develop a new type of catalyst to improve the efficiency of biomass conversion into furan chemicals is of great significance for the industrial application of biomass conversion into high additional energy chemistry, and is also a problem that is urgent to solve in the art.

### SUMMARY

The main objective of the present application is to provide a composite catalyst and a method for synthesizing biobased furan chemicals in order to overcome the shortages in the prior art.

In order to achieve the above objective, the present application provides the following technical solution.

One aspect of the present invention provides a composite catalyst, comprising a first component comprising tetravalent metal salts and a second component comprising acidic metal salts.

Another aspect of the present application provides use of the composite catalyst in a reaction where biobased furan chemicals are prepared from biomass carbohydrates.

Further aspect of the present application provides a method for synthesizing biobased furan chemicals, comprising: at least mixing biomass carbohydrates, a solvent and the composite catalyst to form a mixed reaction system, and reacting the mixed reaction system at 80-110°C, so as to obtain the biobased furan chemicals.

Compared with the prior art, the present application at least has the following advantages:
(1) the provided composite catalyst is simple in component, cheap and easily available;
(2) in the provided method for synthesizing the biobased furan chemicals, the biomass carbohydrates can be simply and efficiently catalyzed into 5-hydroxymethylfurfural under mild conditions, so the method is high in target product yield and has good industrial application prospect.

### BRIEF DESCRIPTION OF THE DRAWINGS

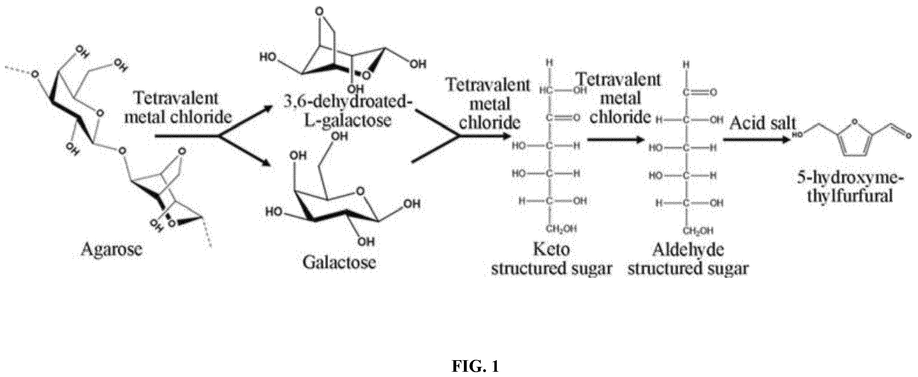

FIG.1 is a process principle diagram of a method for synthesizing biobased furan chemicals in an embodiment of the present application.

### DETAILED DESCRIPTION OF THE EMBODIMENTS

In view of the shortcomings of the existing technology, the applicant has been able to propose the technical solution of the present application through long-term research and practice. The main purpose is to provide a composite metal salt catalyst and a method for synthesizing biobased furan chemicals using this catalyst. The method can efficiently catalyze the conversion of biomass carbohydrates to 5-hydroxymethylfurfural under mild conditions. Next, the technical solution of the present application will be illustrated in detail.

A composite catalyst provided in some embodiments of the present application comprises a first component comprising tetravalent metal salts and a second component comprising acidic metal salts.

In one embodiment, the tetravalent metal salts include tetravalent metal chlorides, for example any one or a combination of more of titanium chloride, zirconium chloride, hafnium chloride, cerium chloride, germanium chloride and tin chloride, but are not limited thereto.

In one embodiment, the acidic metal salts include monovalent or divalent acidic metal salts, for example, any one or a combination of more of potassium dihydrogen phosphate, dipotassium hydrogen phosphate, potassium bisulfate, potassium monopersulfate and sodium bisulfate, and are not limited thereto.

In one embodiment, a mass ratio of the first component to the second component is 5: 1-1:5, for example any numerical value of 5:1, 5:2, 5:3, 5:4, 1:1, 1:5, 2:5, 3:5 and 4:5.

Some embodiments of the present application further provide use of the composite catalyst, comprising: use of the composite catalyst in a reaction where biobased furan chemicals are prepared from biomass carbohydrates.

In one embodiment, the biomass carbohydrates include seaweed derived polysaccharides, for example, the biomass carbohydrates can include but are not limited to agar or agarose.

In one embodiment, the biobased furan chemicals include but are not limited to 5-hydroxymethylfurfural.

Some embodiments of the present application further provide a method for synthesizing biobased furan chemicals, comprising: converting biomass carbohydrates into biobased furan chemicals in the presence of the composite catalyst and under the condition of proper reaction.

Some embodiments of the present application further provide a method for synthesizing biobased furan chemicals, comprising: mixing biomass carbohydrates, a solvent and the composite catalyst to form a mixed reaction system, and reacting the mixed reaction system at 80-110°C, thereby obtaining the biobased furan chemicals.

In one embodiment, a mass ratio of the biobased furan chemicals to the composite catalyst is 5:1-1:5, for example any numerical value of 5:1, 5:2, 5:3, 5:4, 1:1, 1:5, 2:5, 3:5 and 4:5.

In one embodiment, a mass-to-volume ratio of the biomass carbohydrate to the solvent is 1 g: 5 mL-1 g: 40 mL, for example, any numerical value of 1:5, 1:10, 1:15, 1:20, 1:25, 1:30 and 1:40 (g: mL).

In one embodiment, the solvent includes any one or a combination of more of water, dimethyl sulfoxide or ionic liquid, but is not limited thereto

Further, the ionic liquid is any one or a combination of more of a 1-butyl-3-methylimidazole chloride salt, a 1-butyl-3-methylimidazole bromide salt and a 1-allyl-3-methylimidazole chloride salt, but is not limited thereto.

Further, the solvent includes water and dimethyl sulfoxide or ionic liquid, and a mass ratio of water to dimethyl sulfoxide (DMSO) or ionic liquid is 6: 1-1:6, for example, any one numerical value of 1: 1, 6:1, 3:1, 1:1, 1:3 and 1:6.

In one embodiment, the time of the reaction is 1-720 min, further, preferably 1-360 min.

In one embodiment, the biomass carbohydrates include seaweed derived polysaccharides, for example, any one or a combination of more of agar, conventional agarose, low melting point agarose, low electroosmotic agarose and high strength agarose, and are not limited thereto.

In one embodiment, the biobased furan chemicals include 5-hydroxymethylfurfural.

Next, the present application will be further described in detail in combination with specific embodiments, however, the scope of protection of the present application is not limited to these specific embodiments. Unless otherwise specified, the raw materials used in the following embodiments can be obtained from the market or self-made with reference to literature. The production equipment, testing equipment, and testing methods used can also be common equipment or methods in the art.

**Example 1** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20 (unless otherwise specified, g: mL), the reaction temperature was controlled at 100°C, the time was 30 min, and the reaction mixture was analyzed using high-performance liquid chromatography after the reaction was ended, wherein a yield of 5-HMF was 71%.

**Comparative example 1** A method for synthesizing 5-hydroxymethylfurfural comprised: 50 mg of agarose and SnCl₄ were added into a mixed solvent of 200 µL H₂O and 800 µL DMSO, wherein a mole ratio of SnCl₄ to monosaccharide unit in agarose was 0.1:1, the above materials reacted for 60 min at 140°C, and a sample was tested using high-performance liquid chromatography after the reaction was ended, wherein the total yield of 5-HMF and acetylpropionic acid (LA) was 32%, the yield of 5-HMF was 24%, and the yield of LA was 8%.

**Comparative example 2** A method for synthesizing 5-hydroxymethylfurfural comprised:
(1) conventional agarose and a synthesized carbon-based solid acid were evenly mixed in a mass ratio of 5:1 and then added into a reactor;
(2) a mixed solvent whose mass was 10 times the mass sum of the conventional agarose and the synthesized carbon-based solid acid was added into the reactor and reacted for 3 h at 190°C, wherein the mixed solvent contained dimethyl sulfoxide, γ-pentolactone and water in a mass ratio of 2:6:2;
(3) the reaction was cooled to 20°C after being ended, a product 5-HMF was obtained by vacuum filtration and separation, with a yield of 64%.

Compared with the catalyst in example 1, the preparation process of the catalyst used in comparative example 2 is far more complicated and more expansive, at the same time, the synthesis process of 5-HMF in comparative example 2 is far more complicated than the process in example 1, the used reaction temperature is obviously higher than that in example 1, energy consumption is higher, solvent consumption is larger, and therefore green and environmental requirement cannot be met.

**Comparative example 3** A method for synthesizing 5-hydroxymethylfurfural was basically the same as that in example 1 except that zirconium chloride in the composite catalyst was replaced with aluminum chloride. The final yield of 5-HMF was 38%.

**Comparative example 4** A method for synthesizing 5-hydroxymethylfurfural was basically the same as that in example 1 except that the composite catalyst was replaced with an equal mass of potassium bisulfate. The final yield of 5-HMF was 43%.

**Comparative example 5** A method for synthesizing 5-hydroxymethylfurfural was basically the same as that in example 1 except that the composite catalyst was replaced with an equal mass of zirconium chloride. The final yield of 5-HMF was 45%.

**Example 2** A composite catalyst provided in this example comprised titanium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 100°C, the time was 60 min, and the reaction mixture was analyzed using high-performance liquid chromatography after the reaction was ended, wherein a yield of 5-HMF was 58%.

**Example 3** A composite catalyst provided in this example comprised hafnium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 100°C, the time was 150 min, and the reaction mixture was analyzed using high-performance liquid chromatography after the reaction was ended, wherein a yield of 5-HMF was 57%.

**Example 4** A composite catalyst provided in this example comprised cerium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 100°C, the time was 120 min, and the reaction mixture was analyzed using high-performance liquid chromatography after the reaction was ended, wherein a yield of 5-HMF was 55%.

**Example 5** A composite catalyst provided in this example comprised titanium chloride and potassium dihydrogen phosphate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 50%.

**Example 6** A composite catalyst provided in this example comprised germanium chloride and potassium monopersulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:2, and a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 51%.

**Example 7** A composite catalyst provided in this example comprised germanium chloride and sodium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:2, and a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 53%.

**Example 8** A composite catalyst provided in this example comprised zirconium chloride and sodium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 100°C, the time was 120 min, and the reaction mixture was analyzed using high-performance liquid chromatography after the reaction was ended, wherein a yield of 5-HMF was 65%.

**Example 9** A composite catalyst provided in this example comprised titanium chloride and sodium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 100°C, the time was 90 min, and the reaction mixture was analyzed using high-performance liquid chromatography after the reaction was ended, wherein a yield of 5-HMF was 62%.

**Example 10** A composite catalyst provided in this example comprised stannic chloride and sodium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 100°C, the time was 90 min, and the reaction mixture was analyzed using high-performance liquid chromatography after the reaction was ended, wherein a yield of 5-HMF was 60%.

**Example 11** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 73%.

**Example 12** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 110°C, the time was 60 min, and the reaction mixture was analyzed using high-performance liquid chromatography after the reaction was ended, wherein a yield of 5-HMF was 63%.

**Example 13** A composite catalyst provided in this example comprised zirconium chloride and potassium bisulfate in a mass ratio of 1:5.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 64%.

**Example 14** A composite catalyst provided in this example comprised zirconium chloride and potassium bisulfate in a mass ratio of 2:5.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 62%.

**Example 15** A composite catalyst provided in this example comprised zirconium chloride and potassium bisulfate in a mass ratio of 3:5.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 57%.

**Example 16** A composite catalyst provided in this example comprised zirconium chloride and potassium bisulfate in a mass ratio of 4:5.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 52%.

**Example 17** A composite catalyst provided in this example comprised zirconium chloride and potassium bisulfate in a mass ratio of 1:1.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 70%.

**Example 18** A composite catalyst provided in this example comprised zirconium chloride and potassium bisulfate in a mass ratio of 5:4.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was controlled 60 min, and the reaction mixture was analyzed using high-performance liquid chromatography after the reaction was ended, wherein a yield of 5-HMF was 67%.

**Example 19** A composite catalyst provided in this example comprised zirconium chloride and potassium bisulfate in a mass ratio of 5:3.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 65%.

**Example 20** A composite catalyst provided in this example comprised zirconium chloride and potassium bisulfate in a mass ratio of 5:2.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 61%.

**Example 21** A composite catalyst provided in this example comprised zirconium chloride and potassium bisulfate in a mass ratio of 5:1.

A method for synthesizing 5-hydroxymethylfurfural (5-HMF) provided in this example comprised: agarose and a composite catalyst were added into water, wherein a mass ratio of agarose to composite catalyst was 1:3, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 56%.

**Example 22** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:5, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 70%.

**Example 23** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 2:5, and a mass-to-volume ratio of agarose to solvent was 1:20, the reaction temperature was controlled at 95°C, the time was 60 min, and the reaction mixture was analyzed using high-performance liquid chromatography after the reaction was ended, wherein a yield of 5-HMF was 68%.

**Example 24** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 3:5, and a mass-to-volume ratio of agarose to solvent was 1:10, the reaction temperature was controlled at 95°C, the time was 60 min, and the reaction mixture was analyzed using high-performance liquid chromatography after the reaction was ended, wherein a yield of 5-HMF was 65%.

**Example 25** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 4:5, and a mass-to-volume ratio of agarose to solvent was 1: 15 AM, the reaction temperature was controlled at 95°C, the time was 60 min, and the reaction mixture was analyzed using high-performance liquid chromatography after the reaction was ended, wherein a yield of 5-HMF was 55%.

**Example 26** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 1:1, and a mass-to-volume ratio of agarose to solvent was 1:25, the reaction temperature was controlled at 95°C, the time was 60 min, and the reaction mixture was analyzed using high-performance liquid chromatography after the reaction was ended, wherein a yield of 5-HMF was 60%.

**Example 27** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 5:2, and a mass-to-volume ratio of agarose to solvent was 1:30, the reaction temperature was controlled at 95°C, the time was 60 min, and the reaction mixture was analyzed using high-performance liquid chromatography after the reaction was ended, wherein a yield of 5-HMF was 55%.

**Example 28** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 5:3, and a mass-to-volume ratio of agarose to solvent was 1:35 AM, the reaction temperature was controlled at 95°C, the time was 60 min, and the reaction mixture was analyzed using high-performance liquid chromatography after the reaction was ended, wherein a yield of 5-HMF was 58%.

**Example 29** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 5:3, and a mass-to-volume ratio of agarose to solvent was 1:40, the reaction temperature was controlled at 95°C, the time was 60 min, and the reaction mixture was analyzed using high-performance liquid chromatography after the reaction was ended, wherein a yield of 5-HMF was 59%.

**Example 30** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into water, wherein a mass ratio of agarose to composite catalyst was 5:4, and a mass-to-volume ratio of agarose to solvent was 1:40, the reaction temperature was controlled at 95°C, the time was 60 min, and the reaction mixture was analyzed using high-performance liquid chromatography after the reaction was ended, wherein a yield of 5-HMF was 53%.

**Example 31** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of agarose to DMSO in the composite solvent was 1:6 and a mass ratio of agarose to composite catalyst was 1:3, a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 75%.

**Example 32** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of agarose to DMSO in the composite solvent was 1:3 and a mass ratio of agarose to composite catalyst was 1:3, a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 68%.

**Example 33** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of agarose to DMSO in the composite solvent was 1:1, a mass ratio of agarose to composite catalyst was 1:3, a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 57%.

**Example 34** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of agarose to DMSO in the composite solvent was 6:1 and a mass ratio of agarose to composite catalyst was 1:3, and a mass-to-volume ratio of agarose to solvent was 1:20, the reaction temperature was controlled at 95°C, the time was 30 min, and the reaction mixture was analyzed using high-performance liquid chromatography after the reaction was ended, wherein a yield of 5-HMF was 52%.

**Example 35** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of water to 1-butyl-3-methylimidazole chloride salt in the composite solvent was 1:6 and a mass ratio of agarose to composite catalyst was 1:3, a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 70%.

**Example 36** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of water to 1-butyl-3-methylimidazole chloride salt in the composite solvent was 1:3 and a mass ratio of agarose to composite catalyst was 1:3, a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 50%.

**Example 37** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of water to 1-butyl-3-methylimidazole chloride salt in the composite solvent was 6:1 and a mass ratio of agarose to composite catalyst was 1:3, and a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 48%.

**Example 38** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of water to 1-butyl-3-methylimidazole bromide salt in the composite solvent was 1:6 and a mass ratio of agarose to composite catalyst was 1:3, a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 71%.

**Example 39** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of water to 1-allyl-3-methylimidazole chloride salt in the composite solvent was 1:6 and a mass ratio of agarose to composite catalyst was 1:3, and a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 65%.

**Example 40** A composite catalyst provided in this example comprised zirconium chloride and potassium bisulfate in a mass ratio of 1:2.

A method for synthesizing 5-hydroxymethylfurfural (5-HMF) provided in this example comprised: agarose and a composite catalyst were added into a composite solvent, wherein a mass ratio of water to 1-allyl-3-methylimidazole chloride salt in the composite solvent was 1:4 and a mass ratio of agarose to composite catalyst was 1:3, a mass-to-volume ratio of agarose to solvent was 1:20, the reaction temperature was controlled at 100°C, the time was 30 min, and the reaction mixture was analyzed using high-performance liquid chromatography after the reaction was ended, wherein a yield of 5-HMF was 63%.

Although the present application has been described with reference to illustrative embodiments, those skilled in the art will understand that various other changes, omissions, and/or additions may be made without departing from the spirit and scope of the present application, and substantial equivalents may be used to replace the substance of the embodiments. In addition, many modifications can be made without departing from the scope of the present application to adapt specific situations or materials to the teachings of the present application. Therefore, this article is not intended to limit the present application to the specific embodiments disclosed for executing the present application, but rather to include all embodiments within the scope of the attached claims.

## Claims

1. A composite catalyst, comprising a first component comprising tetravalent metal salts and a second component comprising acidic metal salts.

2. The composite catalyst according to claim 1, wherein the tetravalent metal salts comprise tetravalent metal chlorides, and the acidic metal salts comprise monovalent or divalent acidic metal salts.

3. The composite catalyst according to claim 1 or 2, wherein the tetravalent metal chlorides comprise any one or a combination of more of titanium chloride, zirconium chloride, hafnium chloride, cerium chloride, germanium chloride and tin chloride; and/or, the acidic metal salts comprise any one or a combination of more of potassium dihydrogen phosphate, dipotassium hydrogen phosphate, potassium bisulfate, potassium monopersulfate and sodium bisulfate.

4. The composite catalyst according to claim 1, wherein a mass ratio of the first component to the second component is 5:1-1:5.

5. Use of the composite catalyst according to any one of claims 1-4, comprising: applying the composite catalyst to a reaction where biobased furan chemicals are prepared from biomass carbohydrates.

6. The use according to claim 5, wherein the biomass carbohydrates comprise seaweed derived polysaccharides, preferably agar or agarose; the biobased furan chemicals comprise 5-hydroxymethylfurfural.

7. A method for synthesizing biobased furan chemicals, comprising: at least mixing biomass carbohydrates, a solvent and the composite catalyst according to any one of claims 1-4 to form a mixed reaction system, and reacting the mixed reaction system at 80-110°C, so as to obtain the biobased furan chemicals.

8. The method for synthesizing biobased furan chemicals according to claim 7, wherein a mass ratio of the biomass carbohydrate to the composite catalyst is 5:1-1:5.

9. The method for synthesizing biobased furan chemicals according to claim 7, wherein a mass-to-volume ratio of the biomass carbohydrate to the solvent is 1 g: 5 mL: 1 g: 40 mL.

10. The method for synthesizing biobased furan chemicals according to claim 7, wherein the time of the reaction is 1-720 min.

11. The method for synthesizing biobased furan chemicals according to claim 7, wherein the biomass carbohydrates comprise seaweed derived polysaccharides.

12. The method for synthesizing biobased furan chemicals according to claim 11, wherein the biomass carbohydrates comprise agarose.

13. The method for synthesizing biobased furan chemicals according to claim 7, wherein the biobased furan chemicals comprise 5-hydroxymethylfurfural.

14. The method for synthesizing biobased furan chemicals according to claim 7, wherein the solvent comprises any one or a combination of more of water, dimethyl sulfoxide or an ionic liquid.

15. The method for synthesizing biobased furan chemicals according to claim 14, wherein the solvent comprises water and dimethyl sulfoxide or ionic liquid in a mass ratio of 6:1-1:6.
